# FASCICULE DE BREVET EUROPEEN

(11) **EP 0 782 702 B1**
(45) Date de publication et mention de la délivrance du brevet: **13.01.1999**
(21) Numéro de dépôt: 95931262.0
(22) Date de dépôt: 19.09.1995
(51) Int. Cl.: G01N 33/52, A61B 10/00, C12Q 1/28

(54) **DISPOSITIF A USAGE UNIQUE D'ANALYSE D'UN LIQUIDE CORPOREL**
VORRICHTUNG FÜR EINMALIGEN GEBRAUCH ZUR ANALYSE EINER KÖRPERLICHEN FLÜSSIGKEIT
SINGLE-USE BODY FLUID ANALYSIS DEVICE

(30) Priorité: 19.09.1994 FR 9411349
(43) Date de publication de la demande: 09.07.1997
(73) Titulaire: Chaffringeon, Bernard, 1025 Saint-Sulpice (CH)
(72) Inventeur: Chaffringeon, Bernard, 1025 Saint-Sulpice (CH)
(74) Mandataire: Guerre, Dominique
(86) Numéro de dépôt international: FR9501199
(87) Numéro de publication internationale: WO9609544

(56) Documents cités:
- EP-A- 0 166 933
- EP-A- 0 228 752
- EP-A- 0 363 196
- WO-A-91/09309
- US-A- 3 850 160
- US-A- 4 227 537
- US-A- 4 257 427
- US-A- 4 614 715

## Description

La présente invention concerne l'analyse d'un liquide corporel, prélevé directement dans une cavité intracorporelle allongée. A titre d'exemple non limitatif, la présente invention sera introduite, définie et expliquée par rapport au recueil de la glaire cervicale dans la cavité vaginale de la femme, pour la détection de sa période fertile.

Afin de déterminer les périodes de fertilité de la femme, il est connu et il a été proposé de détecter et suivre la présence et/ou concentration en certains constituants biochimiques ou biologiques de ladite glaire, tels qu'une peroxydase ou un composé présentant une activité peroxydasique, ou tels qu'un mucopolysaccharide ou une glycoprotéine, par l'utilisation de réactifs ou systèmes réactifs colorés, par exemple, dans le premier cas un composé d'oxydoréduction, dont au moins la forme oxydée est colorée, par exemple le gaïacol, et dans le second cas la safranine.

Pour la mise en oeuvre de tels réactifs ou systèmes réactionnels, on a généralement proposé des solutions sommaires, et en pratique difficilement mises en oeuvre par la femme, à savoir :
- le prélèvement in situ de la glaire cervicale, par un élément susceptible de recueillir la glaire, tel qu'un écouvillon, puis la mise en contact dudit élément, à l'extérieur de la cavité vaginale, avec le réactif sous forme liquide ;
- l'introduction dans la cavité vaginale d'un tampon absorbant, imprégné ou enrobé d'une couche de réactif, et l'extraction dudit tampon au bout d'un certain temps, avec traitement de la couche de réactif par un agent développant de coloration.

Ces méthodes sont connues par exemple, par les documents FR-A-2 216 975, EP-A-0 363 196 et EP-A-0 555 109.

Les inconvénients de ces méthodes résultent de la nécessité de traiter l'extrait de la glaire cervicale recueillie de façon extemporanée, afin de révéler la présence éventuelle ou concentration de certains de ses constituants biochimiques.

Par ailleurs, de manière à cultiver directement des micro-organismes, notamment pathogènes, éventuellement présents dans une cavité intracorporelle allongée, on a proposé conformément au mode d'exécution de la figure 3 du document US-C-4 257 427, un dispositif à usage unique comprenant :
- un élément de conduit, en matière plastique transparente, entouré d'une gaine en mousse souple ; cet élément présente à ses deux extrémités, un col relativement étroit et un fond ; il est intrinsèquement suffisamment raide ou rigide selon sa longueur, pour être poussé par son fond et introduit dans la cavité intracorporelle ;
- un moyen de transfert du liquide corporel supposé contenir les micro-organismes, monté au moins pour partie à l'intérieur de l'élément de conduit, s'étendant d'une zone de prélèvement du liquide corporel, à l'extérieur du col, à un point de collecte axiale dudit liquide, situé à l'intérieur de l'élément de conduit, du côté de son fond ; ce moyen consiste en une mèche de fils tressés, dont l'extrémité extérieure effilochée sert au prélèvement , et dont l'extrémité intérieure concentre le liquide corporel prélevé ; un tube intermédiaire assure une étanchéité entre la mèche et le col de l'élément de conduit, de telle sorte que l'intérieur de ce dernier se trouve substantiellement isolé de la cavité intracorporelle dans laquelle ledit élément se trouve introduit ;
- un milieu de culture disposé à l'intérieur de l'élément de conduit, contre son fond.

Ce dispositif est introduit par l'utilisateur dans la cavité intracorporelle, dans laquelle il demeure pendant un temps suffisant pour permettre le recueil du liquide corporel, et la culture des micro-organismes recherchés. Puis, l'utilisateur extrait lui-même le dispositif de la cavité intra-corporelle, et observe le développement ou non de la culture du micro-organisme recherché, au travers de la paroi de l'élément de conduit.

La présente invention a pour objet un dispositif à usage unique, permettant une analyse extemporanée d'un liquide corporel, avec des résultats directement identifiables par l'utilisateur, et ceci sans intervention de ce dernier, autre que l'introduction et l'extraction dudit dispositif de la cavité intracorporelle.

Conformément à la présente invention, on part tout d'abord d'un dispositif à usage unique tel que décrit dans le document US-C-4 257 427, lequel est ensuite spécifiquement modifié de la manière suivante :
- d'une part, la zone de prélèvement du moyen de transfert consiste en une couronne périphérique, supportée dans l'ouverture du col de l'élément de conduit, saillant radialement et à l'extérieur dudit col, et ménageant un passage de communication entre la cavité intracorporelle et l'intérieur dudit élément de conduit ;
- et d'autre part, un moyen réactionnel est monté ou disposé sur l'élément de conduit, de manière visible pour l'utilisateur, et en relation avec le point de collecte axiale, pour recevoir au moins une partie du liquide corporel collecté, et comprend au moins un réactif susceptible de réagir avec au moins un composant du liquide corporel, ou en présence d'une condition biologique ou biochimique dudit liquide, pour donner un produit de réaction, notamment coloré, révélant la présence de ladite condition biologique ou biochimique, ou dudit composant dans ledit liquide corporel, et identifiable directement par l'utilisateur.

Grâce à la présente invention, en particulier le recueil du liquide corporel demeure indépendant des conditions de pression différentielle entre la cavité intracorporelle et l'intérieur de l'élément de conduit, ce qui facilite le transfert du liquide corporel vers le moyen réactionnel. Cet avantage est déterminant lorsque le liquide corporel, par exemple glaire cervicale, est plus ou moins fluide, et/ou plus ou moins abondant.

Préférentiellement, l'élément 2 de conduit est adapté en formes et dimensions, pour être logé et retenu directement, par simple construction, dans la cavité intracorporelle, et sa surface extérieure est choisie pour être biocompatible avec la paroi intérieure de la cavité intracorporelle.

Par "biocompatible", on entend le fait que le contact entre la surface extérieure de l'élément de conduit et la paroi de la cavité intracorporelle ne génère aucune réaction biologique adverse, de type toxique ou allergique par exemple.

Avantageusement, le moyen de transfert du liquide corporel comprend un faisceau d'éléments filiformes, rassemblés à une extrémité selon le point de collecte axiale, et dispersés à l'autre extrémité selon la couronne de prélèvement périphérique, chaque élément filiforme étant apte à transporter le liquide corporel, d'une extrémité extérieure à une extrémité intérieure, par exemple par absorption ou capillarité.

De préférence, les éléments filiformes sont des fils creux, ou comprennent des fibres d'au moins un polymère biocompatible, notamment d'un polymère choisi parmi les polymères synthétiques ou naturels hydrophiles, tels que les polyesters, les polyacrylonitriles, les polycarbonates, les polyéthylènes et polypropylènes, les silicones, les alginates, les mousses de polyuréthanne, ou encore la cellulose ou ses esters, éventuellement imprégnées par un agent fluidifiant du liquide corporel.

Préférentiellement, le moyen réactionnel est disposé à l'intérieur de l'élément de conduit, du côté de son fond fermé, en vis-à-vis du point de collecte axiale du moyen de transfert.

Dans ce cas, le fond de l'élément de conduit est transparent, et le réactif est disposé contre ce fond, et ceci en vis-à-vis du point de collecte axiale du moyen de transfert, de telle sorte que lorsque le produit de réaction est coloré, cette couleur ou absence de couleur peut être vue ou visualisée par l'utilisateur ou l'utilisatrice.

Dans une variante selon l'invention, le fond est au moins partiellement ouvert, et une queue de transfert du liquide corporel, du point de collecte axiale à un élément distal de recueil dudit liquide, traverse le fond par son ouverture, et émerge de l'élément de conduit.

Dans ce cas, la queue de transfert est préférentiellement constituée des mêmes éléments filiformes que pour le moyen de transfert, qui peut éventuellement se prolonger au travers du fond de l'élément de conduit, et le moyen réactionnel peut être le même ou différent, selon que l'on cherche à mettre en évidence un composant ou une condition biologique, ou deux composants ou conditions biologiques différents.

Cette variante à l'avantage d'indiquer la présence ou absence de condition biologique ou composant du liquide corporel, sans que l'utilisateur soit pour autant obligé de retirer le dispositif de la cavité intracorporelle.

Le dispositif d'analyse selon l'invention, ainsi décrit, peut donc être utilisé directement, en particulier sans ajout ultérieur de réactif ou révélateur coloré par l'utilisateur ou l'utilisatrice, et dans ce dernier cas, l'observation de la réaction colorée est immédiate après le retrait du dispositif.

La présente invention est maintenant décrite par référence au dessin annexé, dans lequel :
- la figure 1 représente une vue en coupe d'un dispositif d'analyse à usage unique selon l'invention ;
- la figure 2 représente un ensemble prêt à l'emploi, comprenant un dispositif selon figure 1 avec un système applicateur ;
- la figure 3 représente une vue en coupe d'un dispositif à usage unique, selon une autre variante de l'invention.

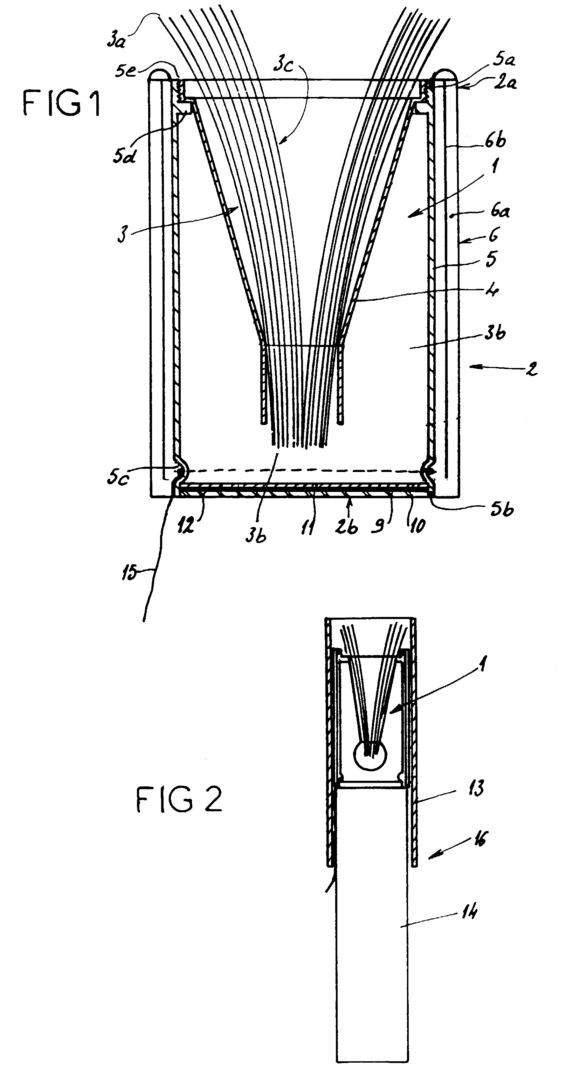

De manière générale, et comme illustré à la figure 1, un dispositif d'analyse 1 à usage unique, de recueil du liquide corporel, par exemple de glaire cervicale, directement dans une cavité intracorporelle allongée, par exemple la cavité vaginale, comprend :
- un élément 2 de conduit, adapté en forme et dimensions pour être logé directement dans, et retenu par simple constriction de la cavité intracorporelle ; cet élément présente respectivement à ses deux extrémités, un col 2a relativement large et un fond 2b ayant la même section transversale ; cet élément est intrinsèquement suffisamment raide ou rigide selon sa longueur, pour être poussé par son fond 2b, et introduit par son col 2a dans la cavité intracorporelle ; et sa surface extérieure est choisie pour être biocompatible avec la paroi intérieure de la cavité intracorporelle ;
- un moyen de transfert 3 du liquide corporel, monté au moins pour partie à l'intérieur de l'élément de conduit 2, et s'étendant d'une couronne ou zone 3a de prélèvement périphérique du liquide corporel, saillant radialement et à l'extérieur du col 2a dudit élément de conduit 2, à un point 3b de collecte axiale dudit liquide, situé à l'intérieur dudit élément de conduit 2, du côté du fond 2b de celui-ci. La couronne 3a est supportée dans l'ouverture du col 2 de l'élément de conduit 2, et ménage un passage par exemple axial, de communication entre la cavité intracorporelle et l'intérieur de l'élément de conduit.

L'élément de conduit 2 peut avoir une structure composite et comprendre un tube 5 relativement rigide, par exemple en matière plastique transparente, avec un col 5a et un fond 5b, sur lequel est éventuellement emmanché un manchon 6 biocompatible, entourant au moins la paroi latérale du tube, constitué par exemple par un tube extérieur 6a en composés d'origine naturelle tels que la cellulose ou la ouate, et une matrice intérieure 6b rigide de support en polymère pour ledit deuxième tube, dont la bordure antérieure (dans le sens de l'introduction du dispositif) est arrondie, et dont la bordure postérieure est à bord droit.

Le moyen de transfert 3 est monté au moins pour partie dans l'élément de conduit 2 ; mais de préférence et comme illustré à la figure 1, le moyen de transfert 3 est monté sur un moyen de support 4 amovible ou fixe, par rapport à l'élément de conduit 2. Plus précisément, le moyen de support 4 a une forme d'entonnoir et est monté sur le tube rigide 5, grâce à un pas de vis 5e et un épaulement 5d prévus sur le tube 5, au niveau de son col 5a.

Le moyen de transfert 3 comprend un faisceau d'éléments filiformes 3c, de préférence des fils creux hydrophiles, rassemblés à une extrémité selon le point 3b de collecte axiale, et dispersés à l'autre extrémité selon la couronne 3a de prélèvement périphérique, chaque élément filiforme 3c assurant le transfert du liquide corporel à recueillir, de l'extérieur du dispositif vers un moyen réactionnel qui sera décrit plus loin, par exemple par absorption ou capillarité.

Ces éléments filiformes 3c peuvent être remplacés par d'autres éléments assurant une migration capillaire ou par tension superficielle du liquide collecté. Toutefois, l'avantage particulier d'utiliser les fils ou fibres est que ces derniers se plient et frottent contre la paroi intracorporelle de la cavité, par la couronne périphérique 3a du moyen de transfert 3, lors de l'insertion du dispositif dans celle-ci, en recueillant ainsi plus de liquide que par simple écoulement sous gravité.

Préférentiellement, on choisit des fibres hydrophiles, car celles-ci acheminent la glaire cervicale, qui est constituée pour sa grande partie par de l'eau, plus rapidement vers le moyen réactionnel, et notamment des fibres de polymères biocompatibles synthétiques ou naturels hydrophiles, tels que les polyesters, les polyacrylonitriles, les polycarbonates, les polyéthylènes et polypropylènes, les silicones, les alginates, les mousses de polyuréthanne, ou encore la cellulose ou ses esters. Ces fibres peuvent être imprégnées avec un liquide fluidifiant ou aidant au transport du liquide corporel.

Un lien d'extraction 15, sous la forme d'un fil ou cordon est fixé à l'élément de conduit 2, du côté du fond 2b, par l'intermédiaire d'une gorge 5c prévue dans le tube 5.

Un moyen ou système réactionnel 9 est disposé à l'intérieur de l'élément de conduit 2, du côté de son fond 2b, en vis-à-vis ou en relation avec le point de collecte axiale 3b du moyen de transfert 3, pour recevoir une partie du liquide corporel collecté. Ce moyen réactionnel incorpore au moins un réactif, et éventuellement un agent fluidifiant pour la glaire cervicale. Ce moyen ou système réactionnel est déposé par exemple soit directement, soit sur une couche absorbante 12. Il est susceptible de réagir avec au moins un composant du liquide corporel recueilli, pour donner au moins un produit de réaction coloré, directement identifiable par l'utilisateur, révélant la présence dudit composant dans le liquide corporel. Comme déjà dit, le réactif coloré sur sa couche absorbante 12 est disposé contre le fond 2b, en vis-à-vis du point de collecte axiale 3b du moyen de transfert 3, et plus précisément contre la paroi de fond transparente 10 du tube 5. La couche 12 imprégnée du réactif 9 est recouverte par un voile 11 hemi-perméable, en ce sens qu'il est perméable vis-à-vis du liquide corporel, dans le sens du transfert, mais qu'il retient ce même liquide dans l'autre sens. Ce voile peut également être imprégné d'un agent fluidifiant pour la glaire cervicale, favorisant le passage des composés que l'on souhaite détecter et étant par exemple un agent tensioactif.

De manière générale, mais comme non représenté à la figure 1, la forme et les dimensions de l'élément 2 de conduit sont adaptées à celles de la cavité vaginale.

Conformément à la figure 2, le dispositif précédemment décrit peut faire partie d'un ensemble prêt à l'emploi, comprenant ce même dispositif 1 et un système applicateur 16. Ce dernier comprend un tube-guide 13, à l'intérieur duquel le dispositif 1 de recueil est inséré. Un poussoir 14 est logé à l'intérieur du tube-guide, en butée contre le dispositif d'analyse. Un tel ensemble peut être introduit directement dans la cavité intracorporelle, par exemple la cavité vaginale, et en poussant le poussoir 14, on libère le dispositif d'analyse 1 par rapport au système applicateur, pour le mettre en place dans cette même cavité.

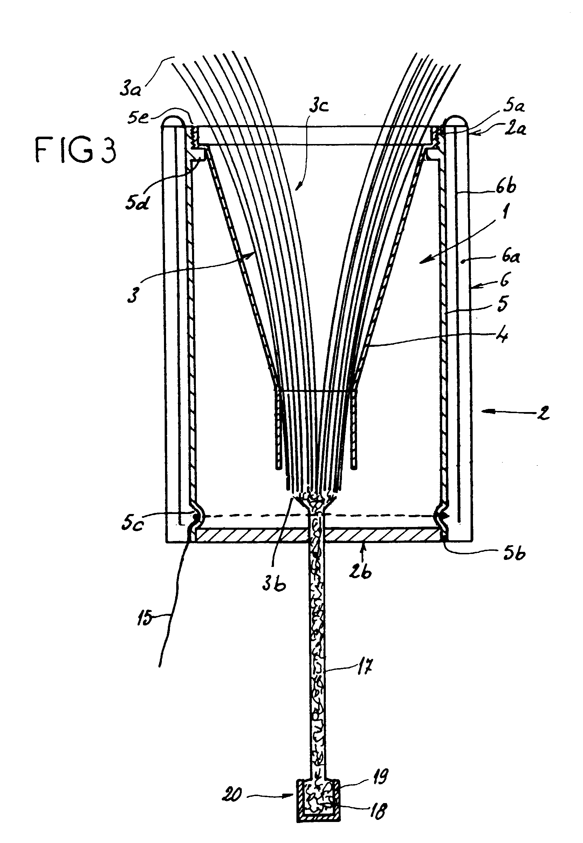

La figure 3 montre une variante de l'invention, selon laquelle le dispositif est essentiellement le même que celui décrit précédemment, avec la différence que le fond 2b est muni d'une ouverture, et une queue 17 de transfert du liquide corporel traverse cette dernière, et émerge de l'élément de conduit 2 vers un élément distal 20 de recueil dudit liquide, muni d'un moyen réactionnel 19.

Ce moyen réactionnel peut être le même ou différent de celui déjà décrit, selon que l'on cherche à mettre en évidence un ou plusieurs composants ou conditions biologiques.

La queue 17, qui peut être une gaine en polyéthylène rigide ou souple ou similaire, comporte également à l'intérieur des éléments filiformes 18, soit les mêmes que pour le dispositif illustré à la figure 1, soit différents, pourvu qu'ils assurent le transfert du liquide corporel du point 3b de collecte axiale vers le moyen réactionnel 19 de l'élément distal 20.

Les dispositifs d'analyse précédemment décrits peuvent ainsi être utilisés, non seulement pour la détection d'un composant dans un liquide corporel, mais aussi pour détecter toute condition chimique, biochimique ou biologique de ce même liquide corporel, à des fins de diagnostic, prophylactiques ou thérapeutiques. Ceci signifie que ce dispositif d'analyse peut incorporer des réactifs ou systèmes réactionnels très différents, de type purement chimique, par exemple enzymatique, ou biologique, par exemple un antigène ou un anticorps, ou encore immuno-enzymatiques.

En conséquence, un dispositif d'analyse selon l'invention peut recevoir de très larges applications, au rang desquelles on peut citer :
- la détection d'une hormone, et notamment d'un pic hormonal ;
- le prélèvement et l'analyse histologique et/ou cytochimique d'un liquide corporel, notamment pour détecter des états pathologiques, ou mettre en évidence certaines périodes physiologiques d'un cycle naturel, par exemple d'un cycle hormonal.

On citera notamment à titre d'exemple pour la détection d'une glycoprotéine ou d'un mucopolysaccharide, et par conséquent, la période fertile chez la femme, les réactifs suivants : la safranine, le bleu de toluidine O, le bleu Alcian, le bleu trypan, un sel de tolonium, le PAS (Schiff périodique acide), la phosphatase alcaline ou un mélange de ceux-ci, éventuellement couplés à un agent favorisant la réaction colorée, tel que le polyvinylpyrrolidone.

## Revendications

1. Dispositif (1) à usage unique d'analyse d'un liquide corporel présent dans une cavité intracorporelle allongée, comprenant :
- un élément (2) de conduit, présentant respectivement à ses deux extrémités un col (2a) et un fond (2b), intrinsèquement suffisamment raide ou rigide selon sa longueur, pour être poussé par son fond (2b) et introduit dans la cavité intracorporelle ;
- un moyen de transfert (3) du liquide corporel, monté au moins pour partie à l'intérieur de l'élément de conduit s'étendant dans une zone (3a) de prélèvement du liquide corporel, à l'extérieur du col (2a), à un point (3b) de collecte axiale dudit liquide, situé à l'intérieur dudit élément de conduit (2), du côté de son fond (2b).
**caractérisé en ce que**, d'une part la zone (3a) de prélèvement du moyen de transfert (3) consiste en une couronne périphérique, supportée dans l'ouverture du col (2) de l'élément (2) de conduit, saillant radialement et à l'extérieur dudit col, et ménageant un passage de communication entre la cavité intracorporelle et l'intérieur dudit élément de conduit, et d'autre part un moyen réactionnel (9,19) est monté sur l'élément de conduit de manière visible pour l'utilisateur, et en relation avec le point (3b) de collecte axiale, pour recevoir au moins une partie du liquide corporel collecté, et comprend au moins un réactif susceptible de réagir avec au moins un composant du liquide corporel, ou en présence d'une condition biologique ou biochimique dudit liquide, pour donner au moins un produit de réaction, notamment coloré, révélant la présence de ladite condition biologique ou biochimique, ou dudit composant dans ledit liquide corporel, et identifiable directement par l'utilisateur.

2. Dispositif selon la revendication 1, caractérisé en ce que l'élément (2) de conduit est adapté en formes et dimensions, pour être logé et retenu directement, par simple construction, dans la cavité intracorporelle, et sa surface extérieure est choisie pour être biocompatible avec la paroi intérieure de la cavité intracorporelle.

3. Dispositif selon la revendication 1, **caractérisé en ce que** le moyen de transfert (3) comprend un faisceau d'éléments filiformes (3c), rassemblés à une extrémité selon le point (3b) de collecte axiale, et dispersés à l'autre extrémité selon la couronne (3a) de prélèvement périphérique, chaque élément filiforme étant apte à transporter le liquide corporel, d'une extrémité extérieure à une extrémité intérieure, par exemple par absorption ou capillarité.

4. Dispositif selon la revendication 3, **caractérisé en ce que** les éléments filiformes (3c) sont des fils creux.

5. Dispositif selon la revendication 3, **caractérisé en ce que** les éléments filiformes (3c) comprennent des fibres d'au moins un polymère biocompatible, notamment d'un polymère choisi parmi les polymères synthétiques ou naturels hydrophiles, notamment les polyesters, les polyacrylonitriles, les alginates, les polycarbonates, les polyéthylènes et polypropylènes, les silicones, les mousses de polyuréthanne, ou encore la cellulose ou ses esters, éventuellement imprégnées par un agent fluidifiant du liquide corporel.

6. Dispositif selon la revendication 1, **caractérisé en ce que** le moyen (9) réactionnel est disposé à l'intérieur de l'élément de conduit (2), du côté de son fond (2b) fermé, en vis-à-vis du point (3b) de collecte axiale du moyen de transfert (3).

7. Dispositif selon la revendication 6, **caractérisé en ce que** le produit de réaction est coloré, le fond (2b) est transparent (10), et le réactif (9) est disposé en couche relativement mince contre le fond (2b), en vis-à-vis du point (3b) de collecte axiale du moyen de transfert (3).

8. Dispositif selon la revendication 7, **caractérisé en ce que** la couche de réactif (9) est recouverte par un voile (11) hemi-perméable, perméable vis-à-vis du liquide corporel, dans le sens du transfert, et retenant ledit liquide dans l'autre sens, et en ce que le voile ou la couche ou les deux sont éventuellement imprégnés d'un agent fluidifiant favorisant le passage des composants à détecter.

9. Dispositif selon l'une quelconque des revendications 1 à 5, **caractérisé en ce que** le fond (2b) est au moins partiellement ouvert, et en ce qu'une queue (17) de transfert du liquide corporel, du point (3b) de collecte axiale à un élément distal (20) de recueil dudit liquide, traverse le fond par son ouverture, et émerge de l'élément de conduit (2).

10. Dispositif selon la revendication 9, **caractérisé en ce que** le moyen (19) réactionnel est associé à l'élément distal (20) de recueil du liquide corporel.

11. Dispositif selon la revendication 1, **caractérisé en ce que** l'élément de conduit (2) comprend un tube (5) relativement rigide avec un col (5a) et un fond (5b), et éventuellement un manchon (6) biocompatible entourant au moins la paroi latérale dudit tube.

12. Dispositif selon la revendication 1, **caractérisé en ce que** le moyen de transfert (3) est monté sur un moyen de support (4) amovible ou fixe par rapport à l'élément de conduit (2).

13. Ensemble prêt à l'emploi, comprenant un dispositif (1) selon la revendication 1 et un système applicateur (16).

## Patentansprüche

1. Vorrichtung zur einmaligen Verwendung für die Analyse einer Körperflüssigkeit, die sich in einem länglichen, intrakorporalen Hohlraum befindet, enthaltend:
- ein Leitelement (2), das an seinen beiden Enden einen Hals (2a) bzw. einen Boden (2b) aufweist und über seine Länge gesehen in sich ausreichend starr bzw. fest genug ist, um über seinem Boden (2b) geschoben und in den intrakorporalen Hohlraum eingeführt zu werden;
- Mittel (3) zum Überbringen der Körperflüssigkeit, die zumindest teilweise im Inneren des Leitelements angeordnet sind und sich von einem Bereich (3a) zum Entnehmen der Körperflüssigkeit außerhalb des Halses (2a) bis zu einem axialen Sammelpunkt (3b) für die Flüssigkeit erstrecken, der sich im Inneren des Leitelements (2) in der Nähe dessen Bodens (2b) befindet,
dadurch gekennzeichnet, daß einerseits der Entnahmebereich (3a) der Überbringmittel (3) aus einem peripheren Kranz besteht, der radial und außerhalb des Halses vorspringt und der in der Öffnung des Halses (2a) des Leitelements (2) gehalten ist und der einen Verbindungsdurchgang zwischen dem intrakorporalen Hohlraum und dem Inneren des Leitelements ausspart, und daß andererseits auf das Leitelement ein Reaktionsmittel (9, 19) für den Anwender sichtbar und in Verbindung mit dem axialen Sammelpunkt (3b) aufgebracht ist, um zumindest einen Teil der gesammelten Körperflüssigkeit zu erhalten, wobei das Reaktionsmittel zumindest ein Reagens umfaßt, das geeignet ist, mit zumindest einem Bestandteil der Körperflüssigkeit oder bei Vorliegen einer biologischen oder biochemischen Beschaffenheit der Flüssigkeit zu reagieren, um zumindest ein vorzugsweise farbiges Reaktionsprodukt zu erzeugen, das das Vorliegen der genannten biologischen oder biochemischen Beschaffenheit oder des Bestandteils in der Körperflüssigkeit für den Anwender direkt identifizierbar anzeigt.

2. Vorrichtung nach Anspruch 1, dadurch gekennzeichnet, daß das Leitelement (2) in seiner Form und seinen Abmessungen derart angepaßt ist, daß es durch einen einfachen Aufbau in den intrakorporalen Hohlraum hineingebracht und direkt gehalten wird, und daß seine Außenseite derart gewählt ist, daß sie mit der Innenwand des intrakorporalen Hohlraums bioverträglich ist.

3. Vorrichtung nach Anspruch 1, dadurch gekennzeichnet, daß die Überbringmittel (3) ein Bündel von fadenförmigen Elementen (3c) aufweisen, die an einem dem axialen Sammelpunkt (3b) entsprechenden Ende zusammenlaufen und die an dem anderen, dem peripheren Aufnahmekranz entsprechenden Ende auseinanderlaufen, wobei jedes fadenförmige Element geeignet ist, die Körperflüssigkeit von einem äußeren Ende zu einem inneren Ende, bspw. durch Absorption oder Kapillarwirkung zu transportieren.

4. Vorrichtung nach Anspruch 3, dadurch gekennzeichnet, daß die fadenförmigen Elemente (3c) Hohlfasern sind.

5. Vorrichtung nach Anspruch 3, dadurch gekennzeichnet, daß die fadenförmigen Elemente (3c) Fasern aus zumindest einem bioverträglichen Polymer aufweisen, insbesondere ein Polymer ausgewählt aus synthetischen oder natürlichen hydrophilen Polymeren, insbesondere Polyester, Polyacrylnitrilen, Alginaten, Polycarbonaten, Polyethylenen oder Polypropylenen, Silikonen, Polyurethanschäumen, oder auch Cellulose oder deren Ester, ggf. imprägniert mit einem Verflüssigungsmittel far die Körperflüssigkeit.

6. Vorrichtung nach Anspruch 1, dadurch gekennzeichnet, daß das Reaktionsmittel (9) im Innern des Leitelements (2) in der Nähe dessen geschlossenen Bodens (2b) und gegenüberliegend zum axialen Sammelpunkt (3b) des Überbringmittels (3) angeordnet ist.

7. Vorrichtung nach Anspruch 6, dadurch gekennzeichnet, daß das Reaktionsprodukt farbig ist, daß der Boden (2b) transparent (10) ist und daß das Reagens (9) in einer relativ dünnen Schicht an dem Boden (2b) gegenüberliegend zum axialen Sammelpunkt (3b) des Überbringmittels (3) angeordnet ist.

8. Vorrichtung nach Anspruch 7, dadurch gekennzeichnet, daß die Reagensschicht (9) mit einer semipermeablen Hülle (11) bedeckt ist, die gegenüber der Körperflüssigkeit in der Transportrichtung durchlässig ist und die die Flüssigkeit in der anderen Richtung zurückhält, und daß die Hülle oder die Schicht oder beide ggf. mit einem Verflüssigungsmittel imprägniert sind, das den Durchgang der zu detektierenden Bestandteile erleichtert.

9. Vorrichtung nach einem der Ansprüche 1 bis 5, dadurch gekennzeichnet, daß der Boden (2b) zumindest teilweise offen ist und daß ein Endstück (17) zum Überbringen der Körperflüssigkeit von dem axialen Sammelpunkt (3b) zu einem distalen Aufnahmeelement (20) für die Flüssigkeit den Boden an seiner Öffnung durchdringt und aus dem Leitelement (2) herausragt.

10. Vorrichtung nach Anspruch 9, dadurch gekennzeichnet, daß das Reaktionsmittel (19) mit dem distalen Aufnahmeelement (20) für die Körperflüssigkeit verbunden ist.

11. Vorrichtung nach Anspruch 1, dadurch gekennzeichnet, daß das Leitelement (2) einen relativ festen Tubus (5) mit einem Hals (5a) und einem Boden (5b) sowie ggf. ein bioverträgliches Überschieberohr (6) umfaßt, das zumindest die Seitenwand des Tubus umgibt.

12. Vorrichtung nach Anspruch 1, dadurch gekennzeichnet, daß das Überbringmittel (3) auf einem bezüglich dem Leitelement (2) unbeweglichen oder festen Trägermittel (4) angebracht ist.

13. Einsatzfähiger Zusammenbau bestehend aus einer Vorrichtung (1) nach Anspruch 1 und einem Applikationssystem (16).

## Claims

1. Disposable device (1) for analysis of a body fluid present in an elongate intracorporeal cavity, comprising:
- a conduit element (2) having respectively, at its two ends, a neck (2a) and a base (2b), inherently sufficiently stiff or rigid along its length for it to be pushed via its base (2b) and introduced into the intracorporeal cavity;
- a means (3) for transferring the body fluid, said means being mounted at least in part in the interior of the conduit element, extending from a zone (3a) for sampling of the body fluid, outside the neck (2a), to an axial collection point (3b) for said fluid, situated inside said conduit element (2), towards its base (2b);
characterized in that, on the one hand, the sampling zone (3a) of the transfer means (3) consists of a peripheral ring, supported in the aperture of the neck (2a) of the conduit element (2), protruding radially and to the outside of said neck, and forming a passage for communication between the intracorporeal cavity and the interior of said conduit element, and, on the other hand, a reaction means (9, 19) is mounted on the conduit element, in a manner visible to the user, and in relation with the axial collection point (3b), in order to receive at least some of the body fluid which has been collected, and it comprises at least one reagent capable of reacting with at least one component of the body fluid, or in the presence of a biological or biochemical state of said fluid, in order to give at least one reaction product, preferably coloured, revealing the presence of said biological or biochemical state, or of said component in said body fluid, and directly identifiable by the user.

2. Device according to Claim 1, characterized in that the conduit element (2) is adapted in shape and dimensions to be fitted and held directly, by simple constriction, in the intracorporeal cavity, and its outer surface is chosen to be biocompatible with the inner wall of the intracorporeal cavity.

3. Device according to Claim 1, characterized in that the transfer means (3) comprises a bundle of filiform elements (3c) which are gathered together at one end at the axial collection point (3b) and are spread out at the other end about the peripheral sampling ring (3a), each filiform element being capable of conveying the body fluid from an outer end to an inner end, for example by absorption or capillarity.

4. Device according to Claim 3, characterized in that the filiform elements (3c) are hollow filaments.

5. Device according to Claim 3, characterized in that the filiform elements (3c) comprise fibres of at least one biocompatible polymer, in particular a polymer chosen from among the hydrophilic natural or synthetic polymers, in particular polyesters, polyacrylonitriles, alginates, polycarbonates, polyethylenes and polypropylenes, silicones, polyurethane foams, or else cellulose or its esters, optionally impregnated with an agent liquefying the body fluid.

6. Device according to Claim 1, characterized in that the reaction means (9) is arranged inside the conduit element (2), at its closed base (2b), facing the axial collection point (3b) of the transfer means (3).

7. Device according to Claim 6, characterized in that the reaction product is coloured, the base (26) is transparent (10), and the reagent (9) is arranged in a relatively thin layer against the base (2b), facing the axial collection point (3b) of the transfer means (3).

8. Device according to Claim 7, characterized in that the layer of reagent (9) is covered by a web (11) which is semipermeable, in the sense that it is permeable with respect to the body fluid in the direction of transfer, while it holds back said fluid in the other direction, and in that the web or the layer or both are optionally impregnated with a liquefying agent promoting the passage of the components which are to be detected.

9. Device according to any one of Claims 1 to 5, characterized in that the base (2b) is at least partially open, and in that a stem (17) for transfer of the body fluid, from the axial collection point (3b) to a distal element (20) for recovery of said fluid, passes through the base via its aperture and emerges from the conduit element (2).

10. Device according to Claim 9, characterized in that the reaction means (19) is associated with the distal element (20) for recovery of the body fluid.

11. Device according to Claim 1, characterized in that the conduit element (2) comprises a relatively rigid tube (5) with a neck (5a) and a base (5b), and if appropriate a biocompatible sleeve (6) surrounding at least the side wall of said tube.

12. Device according to Claim 1, characterized in that the transfer means (3) is mounted on a support means (4) which is removable or fixed in relation to the conduit element (2).

13. Assembly ready for use, comprising a device (1) according to Claim 1 and an applicator system (16).
